# EUROPEAN PATENT APPLICATION

(11) **EP 2 597 463 A1**
(43) Date of publication of application: **29.05.2013**
(21) Application number: 11190071.8
(22) Date of filing: 22.11.2011
(51) Int. Cl.: G01N 33/569, G01N 33/50, C12N 5/0783

(54) **Methods of enrichment and isolation of regulatory T-cells and use of the same**

(71) Applicant: Medizinische Hochschule Hannover, 30625 Hannover (DE)
(72) Inventor: Jäckel, Elmar, Dr. med., 30179 Hannover (DE); Noyan, Fatih, Dr. rer. nat., 30163 Hannover (DE)
(74) Representative: Kröncke, Rolf

(57) **Abstract**

In a first aspect, the present invention relates to a method for the enrichment or purification of regulatory T-cells (T_{regs}). In particular, the method is based on the finding that regulatory T-cells can be separated from other types of T-cells based on the expression of the marker molecule CD154. Moreover, the present invention provides isolated population of T_{regs} cells and kits for enrichment or isolating the same. Said enriched or isolated T-cell population may be used in preventing or treating autoimmune disorders or may be used for preventing or treating transplant rejection or graft-versus-host diseases in a mammal.

## Description

In a first aspect, the present invention relates to a method for the enrichment or purification of regulatory T-cells (T_{regs}). In particular, the method is based on the finding that regulatory T-cells can be separated from other types of T-cells based on the expression of the marker molecule CD154. Moreover, the present invention provides isolated population of in particular antigen-specific and highly pure T_{regs} and kits for enrichment or isolating the same. Said enriched or isolated T-cell population may be used in preventing or treating autoimmune disorders or allergies, or may be used for preventing or treating transplant rejection or graft-versus-host diseases in a mammal.

### Prior art

Organ transplantation offers the possibility of replacing injured or diseased tissue with corresponding organ transplants. The treatment of organ recipients is associated with a lifelong immunosuppressive therapy to prevent allograft rejection. While the immunosuppression can mostly prevent an acute graft rejections chronic graft dysfunction remains a problem difficult to control. Besides this the lifelong use of unspecific immunosuppressive drugs is accompanied by substantial side effects such as infections, worsening renal function, increase of cardiovascular risk and development of malignancies.

Likewise autoimmune diseases such as diabetes, systemic lupus, rheumatoid arthritis, autoimmune hepatitis or multiple sclerosis require life-long immunosuppression with substantial side effects.

Finally patients after allogeneic stem cell transplantation can develop life threatening graft versus host disease, which is often difficult to control by immunosuppressive drugs. Therefore, new ways to induce immune tolerance with less side effects are desperately needed.

The importance of regulatory T cells (Treg) and their immunomodulatory role were subject of research for many years (Sakaguchi, S. et al., Nat Rev Immunol 10, 490―500 (2010). In various experimental rodent models it was shown that T_{regs} possess an important role for the induction and maintenance of immune tolerance. Simultaneously, regulatory T cells have emerged as a promising tool for a cell based clinical application in organ transplantation, e.g. Wood, K.J. & Sakaguchi, S. Nat Rev Immunol 3, 199―210 (2003). Various clinical studies are presently underway (http://www.clinicaltrials.gov) to evaluate the impact of adoptive Treg transfer in the treatment of graft-versus-host-disease (GvHD) posthematopoietic stem cell transplantation (HSCT) and autoimmune diseases such as diabetes. Today gold standard to isolate T_{regs} for use in patients is based on the separation of CD4+CD25high cells by magnetic beads. This protocol is just achieving a purity of about 40-60% of FOXP3+ T_{regs}. This purity may be sufficient to control GvH in lymphopenic patients after stem cell transplantation (Peters, J.H. et al. PLoS ONE 3, e3161 (2008). But in patients, such as solid organ recipients or those who suffer from an autoimmune disease, the infusion of non-regulatory lymphocytes might intensify the disease process.

A major obstacle for the use of human regulatory T cells in cell-based therapy is the lack of an exclusive Treg surface marker for their separation. As already mentioned, the isolation of CD4+CD25high T_{regs} resulted in an impure Treg fraction, with a high ratio of CD4+ cells with a transient FOXP3 and CD25 expression, with no attribution to the regulatory T cell population, by reason of the absence of all the functional properties of T_{regs}. However, the frequency of CD4+ FOXP3+ cells within these isolated populations decrease over time (Hoffmann, P. et al., Blood 108, 4260―4267 (2006). The outgrowth of these non-regulators accompanied by the reduced regulatory capacity constitutes a major limitation of ex vivo expanded CD4+CD25+ cells.

In addition, it was shown that T_{regs} which can recognize antigens from the tissue they should protect (antigen-specific T_{regs}) are more potent in treating autoimmunity or preventing transplant rejection. In summary antigen-specific T_{regs} can establish tissue specific tolerance without perturbance of the general immunocompentence of the patient. (Peters, J.H., et al., *PLoS ONE* 3, e2233 (2008)).

Various molecules have already been described that apparently possess the feasibility for the identification of antigen-specific T_{regs}. The discovery of potential markers for activated, in particular, antigen-specific T_{regs} shall facilitate the opportunity for the isolation of activated, in particular, antigen-specific T_{regs} from leukapheresis products or peripheral blood mononuclear cells (PBMC) and there subsequent use for the improvement of T_{regs} based clinical implications.

In WO 2010/022341 methods of enriching and using regulatory T-cells are described. In particular, it is identified therein that T_{regs} may be enriched or isolated based on FOXP3 expression. It is noted therein that activated regulatory T-cells may be selected based on the presence of at least one of the following molecules: Latency-Associated peptide (LAP), IL-1 receptor type I (CD121a), IL-1 receptor type II (CD121b) and GARP (LRRC32). It is described that the population of T_{regs} isolated according to the methods described therein may be useful in preventing and treating autoimmune diseases, graft-versus-host disease and transplant rejection.

However, FOXP3 which is described as a well described marker for T_{regs} is an intracellular marker molecule, thus, using the same as a marker for enrichment or purification is not suitable. In addition, the methods in WO 2010/022341 do not describe that LAP or GARP positive T_{regs} are antigen-specific T_{regs}. As described therein, all T_{regs} are activated using a polyspecific stimulus. Hence, LAP or GARP positive T_{regs} could be activated e.g in a bystander way (e.g. by proinflammatory cytokines). In addition WO 2010/022341 does not show that LAP or GARP positive cells can distinguish T_{regs} which were activated specifically by their T cell receptor from other T_{regs} within a complex mix of polyspecific cells.

Hence, there is an ongoing need for providing useful methods for enrichment or isolation or purification of e.g. antigen-specific T_{regs}. In particular, said T_{regs} should be suitable for subsequent use in medicinal applications, like prevention or treatment of autoimmune diseases or for preventing or treatment of transplant rejection or graft-versus-host diseases.

CD154 which is also called CD40 ligand or CD40L is a protein being primarily expressed on activated T-cells. The molecule is a member of the TNF superfamily. CD154 binds to CD40 on antigen presenting cells (APC) which leads to many effects depending on the targeted cell type. CD40L is a well known player in the orchestra of costimulatory molecules useful in inducing and directing immune responses. For example, CD 154 induces activation in APC in association with T-cell receptor stimulation by MHC molecules on the APC. Today three main binding partners are described, CD40, *α* 5 *β* 1 integrin and *α* IIb *β*3. The CD154 molecules are primarily expressed on activated CD4+ T lymphocytes, however, also soluble forms thereof are described. Today CD154 expression has also been found on a wide variety of cells including platelets, mast cells, macrophages, basophils, NK-cells, B lymphocytes as well as non-hematopoietic cells like smooth muscle cells, endothelial cells and epithelial cells.

In EP 1 420 253 B1, the use of CD154 and of CD40/CD154 system inhibitors which block or inhibit the interaction between CD40 and CD154 is disclosed as being useful in *in vitro* detection and/or isolation of antigen specific effector T lymphocytes. The method disclosed therein is based on the observation that activated T-cells generally express CD154 on their surface. The same is described e. g. by Frentsch M., et al., Nature Medicine, 11(10), 1118 ― 1124, 2003. It is described therein that activated antigen specific T helper cells express CD154 on their surface, thus, allowing to identify activated CD4 cells based on the antigen induced CD154 expression.

### Brief description of the present invention

The present inventor aims to provide new methods for the enrichment, purification and isolation of preferably antigen-specific regulatory T-cells (T_{regs}). In particular, it has been recognized that T_{regs} can be separated from other types of T-cells based on the expression of the marker molecule CD 154. That is, while other types of T-cells express CD154, in particular in its activated state, T_{regs} do not express CD154 on its surface, neither in its inactivated nor in its activated state. Hence, based on the expression of the marker molecule CD154 T_{regs} can be separated from other types of T-cells.

It is preferred that the e.g. antige-specific T_{regs} are isolated or enriched from a sample containing other cell types by expression of the marker molecule CD4, latency-associated-peptide (LAP) or glycoprotein-A repetitions predominant (GARP) and, in addition, lack of expression of the marker molecule CD154. T_{regs} are characterized in being CD4 positive, moreover being LAP or GARP positive while being CD154 negative. In particular, when isolating activated T_{regs} from a pool containing other types of activated T-cells, the selection or separation of T_{regs} based on the expression of the marker molecule CD154 is advantageous. It is particularly preferred for the isolation of preferably antigen-specific T_{regs}, that activated T_{regs} are separated whereby the activation is preferably an antigen specific activation.

Herein the expression of Latency Associated peptide (LAP) and glycoprotein A repititions predominant (GARP, or LRRC32) for the isolation of polyspecific and alloantigen specific T_{regs} is demonstrated and superiority is shown compared those cell fractions with today standard CD4+CD25high and CD4+CD25+CD127low cells. Furthermore an outstanding Treg purity while combining CD4+LAP+ T_{regs} with the activation marker CD 154 is achieved and it is shown that those Tregs are functional superior e.g. for the induction of tolerance in humanized mouse models. Noteworthy tolerance is achieved in a very immunogenic model of transplantation with allogeneic cells or tissues. As CD4+CD154-LAP+ and CD4+CD154-GARP+ can control such potent immune responses, it is assumed that they are able to control weaker immune responses e.g. in autoimmunity and allergy. As there are no humanized models of human autoimmune diseases the latter aspect cannot be experimentally proven in the human system. However, it is demonstrated in autoimmune mouse models that antigen-specific T_{regs} can control autoimmunity while polyspecific CD4+CD25+ T_{regs} had a substantially weaker effect effect.

In a further aspect, the invention defines antigen-specific T_{regs} by the expression of the markers LAP or GARP. This is of special importance as antigen-specific Tregs could thus so far not be identified and isolated. Further, it is shown herein that in autoimmune diseases and transplantation these antigen-specific T_{regs} are far more potent than polyspecific T_{regs}. In fact, polyspecific T_{regs} were unable to control autommunity or transplant rejection at all after transfer.

In a further aspect, isolated populations of cells containing T_{regs} based on separation of CD154 negative T-cells are provided. It is particularly preferred that the isolated populations of cells contains at least 80 % T_{regs}, in particular 90 % T_{regs}. In a particularly preferred embodiment, the isolated population of cells contains antigen specific T_{regs}.

In addition, a kit for isolating T_{regs}, in particular, activated T_{regs}, like antigen-specific T_{regs} is provided. Said kit comprises means for labelling and separating CD154 positive cells; means for labelling and isolating CD4 positive cells; means for labelling and isolating T-cell expressing LAP and/or GARP and, optionally means for isolating the cells based on the labelling.

Finally, the present invention relates to the use of T_{regs} isolated according to the present invention in preventing or treating autoimmune diseases or allergies in a mammal or for preventing or treatment of transplant rejection or graft-host-disease in a mammal.

### Brief description of the drawings

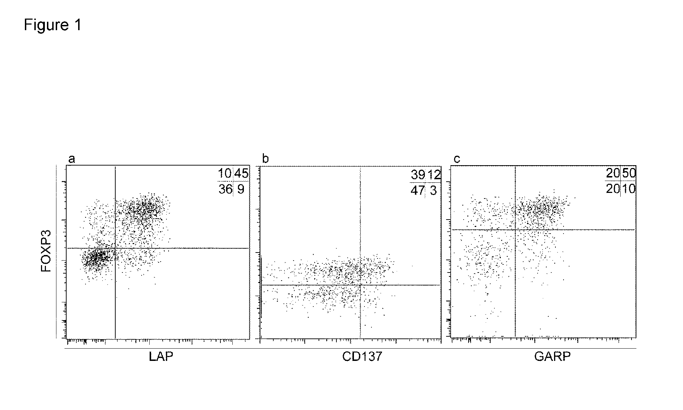

**Figure 1****:** LAP and GARP identify activated human T_{regs} (Analysis of CD4⁺CD25^{high} cells). Flow cytometric analysis of activation markers on polyspecific activated CD4⁺CD25^{high} cells. The sensitivity to detect activated FOXP3⁺ cells is 82% for LAP (a), 23% for CD137 (b) and 71% for GARP (d). On the other hand the specificity to identify FOXP3⁺ cells is 83% for LAP⁺ cells (a), 80% for CD137⁺ cells (b) and 83% for GARP⁺ cells (c).

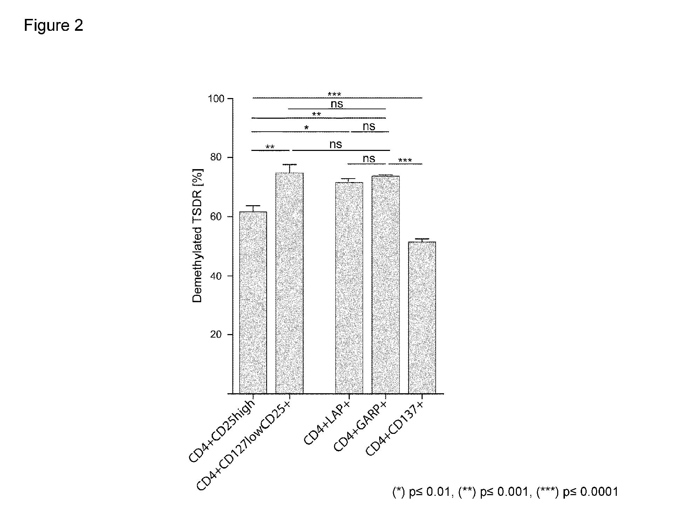

**Figure** 2: **LAP identifies human** T_{regs} **as analyzed by TSDR demethylation.** Methylation Analysis of TSDR of sorted CD4⁺CD25^{high}, CD4⁺CD25⁺CD12^{low}, CD4⁺CD137⁺ and CD4⁺LAP⁺. The purity of CD4+LAP+ and CD4+GARP+ regulatory T cells based on TSDR (Treg specific demethylation region) demethylation is as high as in CD4⁺CD25⁺CD127^{low} cells and significantly higher than in CD4⁺CD25^{high} sorted cells. In contrast the Treg purity of CD4⁺CD137⁺ cells is inferior to CD4⁺CD25^{high} and CD4⁺CD25⁺CD127^{low} cells. Besides purity LAP⁺ and CD137⁺ cells are antigen-specific compared to the CD4⁺CD25^{high} and CD4⁺CD25⁺CD127^{low} cells.

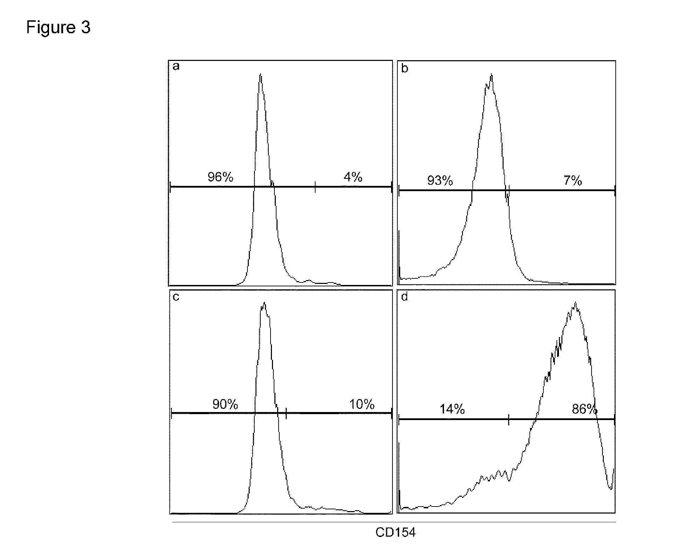

**Figure 3****: CD154 expression is not seen on resting or activated** T_{regs} **and distinguishes activated** T_{regs} **from other activated T cells.** FACS analysis revealed that CD154 is neither expressed on a) rested CD4⁺CD25⁺CD127^{low} nor on b) activated CD4⁺CD25⁺CD127^{low} cells. While resting T cells do not express CD154 (c), activated T cells express CD154 in 86% (d).

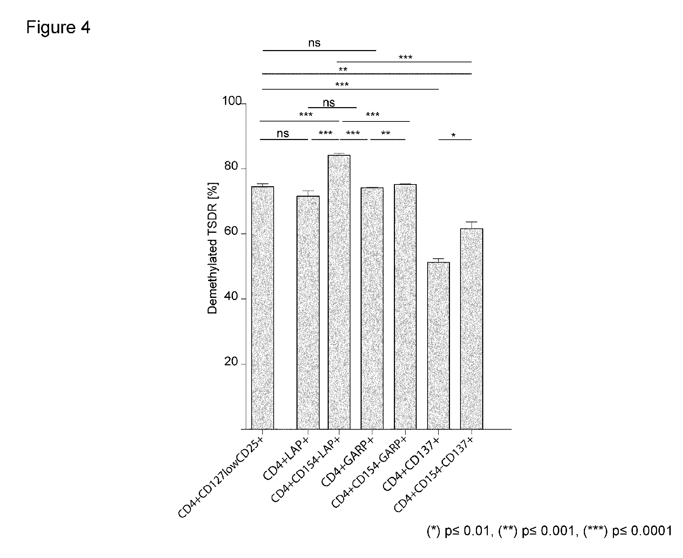

**Figure 4****: CD154- LAP+ identifies human T_{regs} with high purity as analyzed by TSDR demethylation.**
   Methylation analysis revealed that the implementation of the additional surface marker CD154 increases significantly the purity of isolated activated, antigen-specific T_{regs}. While the proportion of demethylated cells isolated from CD4⁺LAP⁺ CD4+GARP+ population was as comparable to CD4⁺CD25⁺CD127^{low}, the addition of CD154 negativity resulted in a significant higher purity of CD4⁺CD154⁻LAP⁺ and CD4+CD154-GARP+ compared to CD4+ LAP+, CD4+GARP+ and CD4⁺CD25⁺CD127^{low}cells. However the latter are non-antigen-specific and cannot be obtained by magnetic separation.

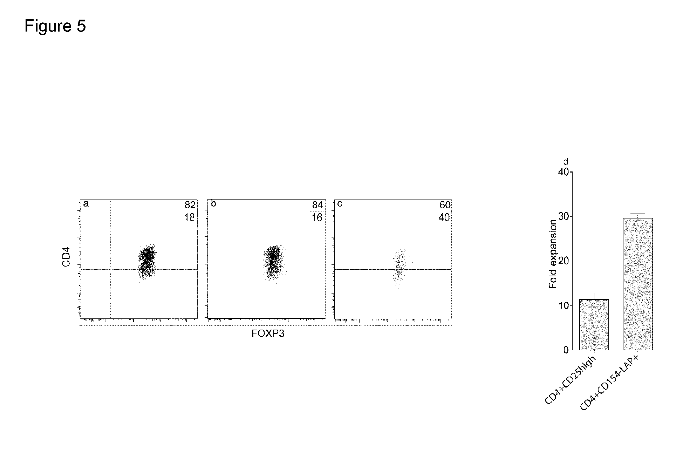

**Figure 5****: Isolated CD4+, CD154⁻, LAP⁺** T_{regs} **can be efficiently expanded and remain highly pure.**
   CD4+, CD154-, LAP+ T_{regs} were isolated after initial polyspecific activation and expanded with Dynabeads-Human Treg Expander (Invitrogen)in the presence of IL-2. a) CD4⁺CD154⁻LAP⁺ showed a stable expression of FOXP3 over prolonged periods of expansion (21 days). The purity was comparable to the expansion of b) CD4⁺CD25⁺CD127^{low} cells while c) CD4⁺CD25^{high} significantly lost FOXP3 expressing cells. d) Fold expansion of CD4⁺CD154⁻LAP⁺ was up to three times higher compared to fold expansion of CD4⁺CD25^{high} cells.

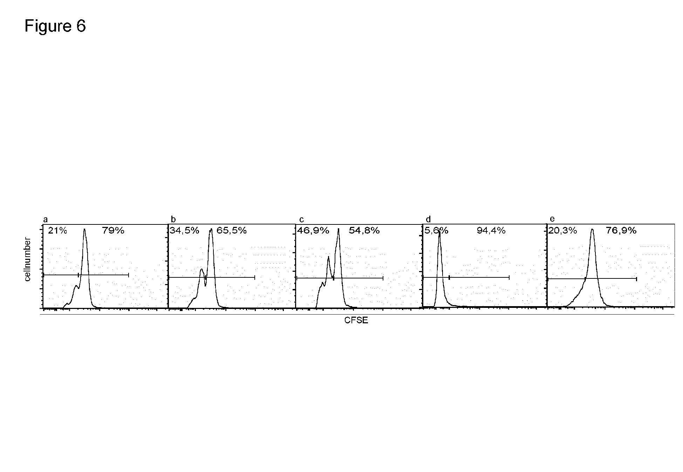

**Figure 6****: CD4+, CD154⁻_{,} LAP⁺** T_{regs} **are functionally superior to currently used standard isolations of** T_{regs}.
   *In vitro* co-culture assays of T_{regs} with naive Teffs (1:1 ratio) revealed that (a) CD4⁺CD154⁻LAP⁺ T_{regs} were more potent in the inhibition of proliferation of Teffs compared to (b) CD4⁺CD25⁺CD127^{low} and (c) CD4⁺CD25^{high} T_{regs}. (d) represented CFSE based measurement of naive CD8+ and (e) of activated CD8+ cell proliferation.
**Figure 7****: Reconstitution of NOD RAG1**⁻**^{/-}γc^{-/-} mice with human PBMCs leads to a reconstitution with human T cells.**
   Two weeks after reconstitution with human PBMCs, FACS analysis showed a reconstitution with CD4+ and CD8+ T cells (a). Spleenocytes from reconstituted humanized mice were polyclonal activated and accordingly activated T_{regs} could be identified by their LAP expression (b).
**Figure 8****: CD154⁻, LAP⁺ T_{regs} prevent rejection of allogeneic cells in reconstituted humanized mice.**
   a) PBMCs from syngeneic (high CFSE) and allogeneic (low CFSE) donors were marked with different intensities of CFSE. b) Allogeneic target cells were efficiently killed in immune reconstituted mice, while syngeneic targets were not affected. Allospecific CD154⁻LAP⁺ T_{regs} completely inibited the killing of allospecific taregets (c) while polyspecifc CD154⁻LAP⁺ T_{regs} just partially prevented the killing of allospecific targets (d).

### Detailed description of the present invention

The present inventors aim in providing a new method for the enrichment, isolation or purification of preferably antigen-specific regulatory T-cells. Said method comprises the step of providing a sample containing T-cells from a mammal; and separating the regulatory T-cells from other types of T-cells based on the expression of the marker molecule CD154.

That is, the present inventors recognized that CD154 allows to differentiate between CD4 positive helper cells and CD4 positive T_{regs}. While CD4 positive helper cells express CD154 on its surface, T_{regs} are CD154 negative both in resting as well as in activated stage. This is in sharp contrast to previous reports wherein CD154 has been reported being a good activation marker of activated effector T-cells (CD4 positive cells).

Thus, it is preferred that the T_{regs} are purified by a step of separating said cells from other types of T-cells based on the expression of the marker molecule CD154, i. e. being CD154 negative. In addition, it is preferred that the method comprises the step of enrichment of T-cells based on the expression of the marker molecule CD4. T_{regs} either in an activated as well as in a resting stage are CD4 positive and CD154 negative.

It is particularly preferred that the method according to the present invention comprises further the step of isolating the T_{regs} based on the expression of the marker molecule a.) latency-associated peptide (LAP) or b.) glycoprotein A repetitions predominant (GARP).

In particular, using LAP as a marker molecule allows to separate T_{regs}, like antigen-specific T_{regs} from other T-cell populations. Hence, in a preferred embodiment, the method for the purification of regulatory T-cells is based on purifying T-cells being CD4 positive, LAP positive and CD154 negative. Thus, in particular, the combination of CD154, LAP and, CD4 allows the isolation of highly pure T_{regs}, like antigen-specific T_{regs}.

It is particularly preferred that the T-cells provided in the samples are T-cells being activated in advance. In contrast to other activated T-cells, T_{regs} do not express the CD154 molecule on the surface after activation.

The activation of the T-cells may be specifically or polyspecifically. However, it is preferred that the T-cells are activated antigen specific. That is, in a preferred embodiment, antigen specific T_{regs} are enriched or isolated based on the expression of LAP and CD4 while being negative for CD 154.

Moreover, it is demonstrated herein that LAP represents a suitable marker for identifying antigen specific T_{regs}. Hence, it is particularly preferred that antigen specific T_{regs} are isolated based on the combination of CD4+ and LAP+, preferably of CD4 positive, LAP positive and CD154 negative.

In an alternative embodiment, T_{regs} are isolated based on the combination of being CD4 positive, CD154 negative and GARP positive.

The skilled person is well aware of suitable methods for isolating the T_{regs}. It is preferred, that isolation or separation of the T_{regs} from other cells comprises contacting the sample with a labelled antibody or antibody fragment that specifically recognizes the marker molecules.

It is particularly preferred that the antibody used for separation are labelled monoclonal antibodies or molecules containing a fragment of said monoclonal antibody.

In another embodiment, the present invention relates to the use of the marker molecules LAP and GARP for differentiation between antigen-specific activated T_{regs} and T_{regs} not being activated due to antigen exposure in advance. Hence, LAP and GARP represent suitable marker molecules for identifying and selecting antigen specific T_{regs} from a pool of optionally pre-enriched T-cells, in particular, pre-enriched T_{regs}. Said pre-enriched T_{regs} are for example T_{regs} population isolated or enriched based on CD4+CD25+CD127+ expression or CD4+CD25high expression. LAP and GARP allows to differentiate T-cells into antigen-activated T-cells and T-cells being activated unspecifically by bystander activation.

As used herein, the term "sample" or "biological sample" refers to tissues or body fluids removed from a mammal, preferably a human, containing T-cells. Samples are preferably blood and blood fractions, including peripheral blood. The biological sample drawn from the body of the mammal, such as a human, may be blood, cord blood or similar tissues or cells. Methods for obtaining such samples are well known to workers in the fields of cellular immunology and surgery.

The term "isolated" with regard to a population of cells as used herein refers to a cell population with either has no naturally-occuring counterpart or has been separated or purified from other components including other cell types which naturally accompany it, i.e. in normal or disease tissues or body fluids. Typically, an isolated cell population is at least 2 fold, 4 fold, 8 fold or more enriched for a specific cell type when compared to the natural source from which the population was obtained.

It is particularly preferred, that the isolated population of cells obtainable with the method according to the present invention is a population of cells containing at least 70 %, preferably at least 75 %, like at least 80 %, e.g. at least 90 %, at least 95 % or even more, preferably more than 95 % T_{regs}, in particular antigen specific T_{regs}.

The amount of T_{regs} is established based on the analysis of the methylation status analyzed by quantification of the Treg specific demethylation region (TSDR) as described in (Polansky, JK et al., Eur J Immunol 38(6), 1654-63). Quantification of human Tregs by quantification of their characteristic epigenetic modifications (TSDR) is currently regarded to be the gold standard to assess the purity of Tregs in complex samples.

The phrase "specifically (or selectively) binds" to an antibody or "specifically (or selectively) immunoreactive with "when referring to a protein or peptide, refers to a binding reaction that is determinative of the presence of the protein, often in heterogeneous population of proteins and other biologics. Specific binding to an antibody under such conditions requires an antibody that is selective for its specificity for a particular protein. For example, polyclonal antibodies can be selected to obtain only those polyclonal antibodies that are specifically immunoreactive with the selected antigen and not with other proteins.

A "label" or a "detectable moiety" is covalently or noncovalently attached to the binding moiety specifically binding to the marker molecule. It is preferred that the binding moiety is an antibody. The label may be detectable by spectroscopic, photochemical, biochemical, immunochemical, chemical or other physical means. Particularly useful labels are fluorescence dyes. In addition, a suitable label is a magnetic particle, such as a paramagnetic microbead as used and applied by Miltenyi Biotec, Germany.

That is, separation or enrichment is affected by immunological selection techniques, in particular, wherein the immunological selection technique is at least one of flow cytometry, fluorescence activated cell sorting (FACS), and magnetic bead based isolation techniques.

The skilled person is well aware of suitable methods for separation or enrichment.

It is particularly preferred that the method allows purification of T_{regs} greater than 80 % of the total population of isolated cells, preferably greater than 85 %, or even greater than 90 %, most preferably greater than 95 % whereby the amount of T_{regs} is determined based on TDSR.

As used herein, the terms "isolation", "purification", or "enrichment" are used interchangeably. Unless otherwise indicated, one of these terms includes also the other terms. That is, when referring to purification, said term includes isolation or enrichment, respectively, unless otherwise indicated.

Further, it is not only possible to purify T_{regs} according to CD154 expression but also to analyze a sample from a mammal for the presence of T_{regs} based on the expression of CD154. That is, in another aspect, the present invention relates to a method for analysing a sample from a mammal for the presence of T_{regs} and, optionally, determining qualitatively or quantitatively the amounts of T_{regs} present comprising the step of providing a sample containing T-cells from a mammal at determining T_{regs} based on the expression of the marker molecule CD154 in combination with the marker molecule CD4. It is particularly preferred that the presence of antigen-specific T_{regs} is analyzed based on expression of CD4, CD154 and LAP and/or GARP.

In another aspect, the present invention relates to an isolated population of cells containing T_{regs} in particular, antigen specific T_{regs}. In particular, the isolated population of cells contains at least 80 % T_{regs}, preferably at least 85 % T_{regs}, in particular preferred at least 90 % T_{regs}. The isolated population of cells containing T_{regs} may be administrated for therapeutic purposes to a mammal. In particular, the isolated cell population may be provided in a suitable physiologically acceptable medium allowing administration for therapeutic purposes. In addition, said isolated population of cells according to the present invention may be obtained by *in vitro* expansion of isolated T_{regs} according to the present invention. The skilled person is well aware of suitable methods allowing expansion and cultivation of T_{regs}. In particular, for therapeutic purposes, like adoptive transfer, *in vitro* expansion of the T_{regs} is required to obtain a suitable amount of T_{regs} useful for medicinal purposes.

As demonstrated herein, the T_{regs} according to the present invention obtained according to the method according to the present invention, i.e. CD4+, CD154-, LAP+ T_{regs} can be easily amplified using GMP-conform conditions and IL-2 while maintaining its high purity throughout expansion. This is in contrast to expansion of T_{regs} described in the art as discussed below.

In another aspect of the present invention, kits are provided for practising the method according to the present invention. The kits allow to isolate T_{regs}. That is, the kit according to the present invention for isolating T_{regs}, in particular, activated T_{regs}, comprises means for labelling and separating CD154 positive cells; means for labelling and isolating CD4 positive T-cells; means for labelling and isolating T-cells expressing LAP and/or GARP; and, optionally, means for isolating labelled cells. Typically, said means are antibodies being labelled with a label allowing isolation based on the label. In certain embodiments, the kits include at least antibodies to CD154, CD4 as well as LAP and/or GARP. The means for isolating the labelled cells are adapted for isolation based on the label. For example, when labelling with paramagnetic beads, the means for isolating said label cells is a magnet etc. In addition, the kit may further include instructions for practising the isolation methods of the present invention.

It is preferred, that in the kit according to the present invention means for labelling and isolation the cells are antibodies or comprising antibody fragments specifically recognizing the marker molecules.

Moreover, it is preferred that the label of the means is at least one of a fluorescence, a magnetic or an isotope label.

It is particularly preferred that the kit according to the present invention allows to isolate activated T_{regs}, in particular, antigen specific activated T_{regs}. Hence, it is possible that the kit according to the present invention can contain the antigen for activation of said T-cells.

In another aspect, isolated T_{regs} are provided being isolated or enriched according to a method of the present invention. The T_{regs} according to the present invention may be used in preventing or treating an autoimmune disease or a disorder in a mammal. Typical examples of autoimmune diseases to be treated with the T_{regs} according to the present invention include, but are not limited to systemic lupus erythematosus (SLE), rheumatoid arthritis, Type I diabetes, multiple sclerosis (MS), aplastic anemia, autoimmune thyroid disease, autoimmune hepatitis, multiple endocrine autoimmune syndromes, inflammatory bowel disease, autoimmune polyneuropathy, autoimmune vascultitis and immunodysregulation polyendocrinopathy enteropathy X-linked syndrome (IPEX).

In another aspect, isolated T_{regs} are provided being isolated or enriched according to a method of the present invention. The T_{regs} according to the present invention may be used in preventing or treating allergic disorders in a mammal. Typical examples of allergic diseases to be treated with the T_{regs} according to the present invention include, but are not limited to allergic polynosis, asthma, enteric allergies and allergic skin reactions such as psoriasis and atopic eczema.

In another aspect, the T_{regs} are useful in preventing or treating transplant rej ection.

The physician is well aware of diseases, disorders or conditions where T_{regs} are useful in treatment regimen. The diseases, disorders or conditions which may be treated with the T_{regs} according to the present invention are characterized by altered (relative to the subject not suffering from the disease or disorder) regulatory T-cell activity or overwhelming effector immune responses of the innate or the adaptive immune system against self-antigens, alloantigens or allergens. These subjects may be treated with pharmaceutical compositions or medicaments containing the T_{regs} of the present invention. The T_{regs} of the present invention allow to modulate one or more of the activities of T-cells, B cells or innate immune cells in the individual. In particular, diseases, disorders or conditions involving immunological responses may be regulated.

Typically, the composition containing the T_{regs} according to the present invention are administered intravascular or intratacheal in case of airway hypersensitization.

Of course, the subject being treated with T_{regs} according to the present invention may obtain other active ingredients, e.g. immunosuppressions, simultaneously, separately, or sequentially.

The present invention will be described in further details by a way of example without limiting the invention thereto.

### Methods

### Cell culture

All T-cell cultures and all T-cell related assays were performed in complete media (RPMI1640, with supplements) in humified incubators at 37°C and 5% CO2.

### Purification of PBMCs, T cell activation, cell separation

Peripheral blood mononuclear cells (PBMCs) from healthy donors were separated by density gradient centrifugation over Ficoll-Paque Plus (GE Healthcare).

### Surface marker labeling for FACS or cell separation

For the isolation of CD4+CD25high, CD4+CD25+CD127low cells, PBMCs were first incubated with RPMI 1640 supp. 10% FCS at 37°C twice for 45 minutes to remove adherent cells. Non adherent cells were collected, washed and subsequently labelled with mAb combinations. CD4 (BD, Heidelberg, Clone RPA-T4), CD25 (BD, Heidelberg, Clone M-A251), CD127 (BD, Heidelberg, Clone hIL-7RM21). The FACS based isolation of above mentioned cell populations were performed at cell sorting facility of Medical School Hannover with a purity of >90%. For the analysis of LAP (R&D Systems, USA, Clone 27232), GARP (eBioscience, Frankfurt, Clone G14D9) and CD137 (BD, Heidelberg, Clone 4B4-1) isolated PBMCs or pre-sorted CD4+CD25high cells were polyspecifically activated with plate bound α-CD3 (self-made, 5µg/ml) and soluble α-CD28 (BioLegend, Fell, Clone CD28.2, 5µg/ml) for accordingly 6 - 48hours. FACS analysis, respective FACS based isolation of LAP+, GARP+ and CD137+ cells were performed as already described. For the detection of CD154 (Miltenyi, Bergisch Gladbach, Clone 5c8 expression on T cells we activated PBMCs in the presence of α-CD40 (self-made, 1µg/ml). The labelling of surface epitopes is performed as previously. The intracellular FOXP3 (eBioscience, Frankfurt, Clone150D/E4) labelling was performed according to manufacturers information. For the specific TCR activation with a superantigen isolated CD4+CD25+ cells were activated with the staphylococcal enterotoxin B-1 (provided by B Fleischer, Bernhard Nocht Institut, Hamburg). FACS analysis of TCR-vβ repertoire was performed with IO-Test Beta Mark (Beckman Coulter, Krefeld). Allogeneic activation of PBMCs were performed with unrelated and unmatched PBMCs from healthy donors with various effector to target ratios. Target cells were irradiated (50Gy) and CFSE labelled (Invitrogen, 0.2mM).

### DNA Methylation Analysis

DNA methylation analysis was performed by a bisulfite sequencing (Floess, S. et al. Epigenetic control of the foxp3 locus in regulatory T cells. PLoS Biol. 5, e38 (2007). All epigenetic analyses were accomplished by Fa. Epiontis, Berlin.

### T-cell proliferation assays

CD4+CD154-LAP+, CD4+CD25high and CD4+CD25+CD127low T_{regs} were cocultured in a Teff:Tresp ratio of 4:1 with sorted and CFSE labelled (0.2mM) CD8+ cells for 96 hours in 96-well round-bottom microplate. FACS analysis of CD8+ proliferation was assessed according to CFSE intensity.

### Mice

All animal experiments were approved by the local Ethics Animal Review Board (Oldenburg, Germany) and performed according to current German law regulations. NOD-RAG1^{null}IL2γ^{null} were bred in specific pathogen-free facilities at Hannover Medical School.

### Reconstitution of NOD-RAG1^{null}IL2γ^{null} mice

5*10⁶ PBMCs were intra peritonial injected into NOD-RAG1^{null}IL2γ^{null} mice. For the reconstitution analysis peripheral blood was taken out of the mandibular vene and processed accordingly. The reconstitution of animals was monitored by FACS analysis by the detection of human CD4 and human CD8 positive cells.

### Trans vivo DTH

5*10⁵ PBMCs and 5*10⁵ irradiated allogeneic PBMCs were injected into ear pinnae of not-reconstituted NOD-RAG1^{null}IL2γ^{null} mice using 30-gauge 0.5ml insulin syringes (BD). Ear thickness was measured using a spring-loaded digital thickness gauge before and after 24 hours after injection. For the generation of allospecific CD4+CD154-LAP+ T_{regs}, PBMCs were allogeneic activated with irradiated target cells and isolated 24 hours post-activation, accordingly. Isolated cells were expanded with α-CD3/α-CD28 Dynabeads (Invitrogen) and 600U/ml IL-2 (Proleukin, Chiron) for two rounds. For the generation of polyspecific CD4+CD154-LAP+ T_{regs}, target cells were activated with α-CD3 plate bound (5µg/ml) and soluble α-CD28 (5µg/ml). 24 hours post-activation CD4+CD154-LAP+ were FACS sorted and accordingly expanded. For the assessment of function and role of allogeneic or polyspecific CD4+CD154-LAP+ cells, 5*10⁵ cells were mixed with 5*10⁵ PBMCs and 5*10⁵ irradiated allogeneic PBMCs and injected into ear pinnae of not-reconstituted NOD-RAG1^{null}IL2γ^{null} mice. The analysis of the swelling response was measured as described previously 24 hours post-inj ection.

### In vivo killing assay

NOD-RAG1^{null}IL2γ^{null} mice were reconstituted with 5*10⁶ PBMCs from healthy donor. 14 days post-reconstitution 5*10⁵ CFSE high labelled (5mM) syngeneic PBMCs and 5*10⁵ CFSE low labelled (0.5mM) allogeneic PBMCs were injected into reconstituted animals. Additionally 5*10⁴ poly- or 5*10⁴ allospecific isolated and expanded CD4+CD154-LAP+ T_{regs} were added each to the syn- and allogeneic PBMCs mixture. For the rejection analysis of target PBMCs, spleens were removed 3 hours post-injection and processed. The labelling was performed with human mAbs accordingly.

### Results

### Identification of activated T_{regs}

For the characterization of surface markers of activated T_{regs} the expression of various surface molecules upon activation was compared. Herefore, the identification of previously described potential molecules on activated T_{regs}: LAP and GARP and CD137 (4-1BB) have been investigated. To evaluate the most consistent and useful marker their expression on polyclonally activated presorted CD4+CD25high as well as on freshly isolated and activated human PBMCs was analysed. It has been shown that LAP was upregulated in over 80% (Figure la), CD137 in over 23% (Figure 1b) and GARP in over 70% (Figure 1c) of activated T_{regs}, respectively. However, the eligibility for the identification of activated T_{regs} when isolating T_{regs} from complex cell mixtures such as peripheral blood mononuclear cells (PBMCs) was apparently limited to the expression of LAP and GARP. By use of these marker approximately 70% of LAP cells could be attributed to the FOXP3+ Treg compartment. GARP identified therefore 55% and CD137 only 30% of FOXP3+ T_{regs}. Kinetic studies revealed that LAP, GARP and CD137 were rapidly expressed upon activation with α-CD3/ α-CD28.

### LAP and GARP can be used for the isolation of activated T_{regs}

To validate the FACS data and for further characterization of CD4+LAP+ and CD4+GARP+ the corresponding populations were isolated according to their surface markers from freshly isolated and activated PBMCs. A methylation analysis of the Treg specific demethylated region (TSDR) was performed and compared with today gold standards CD4+CD25high as well as CD4+CD25+CD127low cells. Quantification of of the epigenetic demethylation in the TSDR region of the FOXP3 promotor allows the most accurate quantification of T_{regs} and is regarded to be the gold standard. While induced T_{regs} or transient FOXP3 expressing effector T cells show a methylated FOXP3 locus, "true" T_{regs} can be identified according to their demethylated TSDR. Only 49%±1 of isolated CD4+ CD137+ cells had a demethylated TSDR, whereas the demethylated region of sorted CD4+CD25high with 63%±3 was significantly higher. The TSDR analysis of CD4+LAP+ and CD4+GARP+ cells confirmed the FACS data in respect to their Treg phenotype. It can be shown, that with 72%±1 and 71%±1 purity these sorted CD4+LAP+ and CD4+GARP+ cells were indicated as "true T_{regs}". The rate of purity was significantly higher than of analyzed CD4+CD25high and comparable high with CD4+CD25+CD127low cells. However in contrast to the latter ones CD4+LAP+ or CD4+GARP+ T_{regs} are antigen-specific as outlined below.

### LAP identifies specific activated vβ Treg subsets

Currently it was shown, that LAP is selectively expressed on FOXP3+ regulatory T cells upon polyclonal stimulation with α-CD3 and α-CD28. Based on mAbs combination CD4 and LAP sorting of those activated regulatory T cells resulted in a polyclonal T cell pool with various different TCRs. To elucidate the identification of antigen-specific T_{regs} being activated by their T cell receptor and to exclude cross-activations or bystander-activations the identification of specifically activated TCRs was determined. Therefore, the sorted CD4+CD25high cells were stimulated with a superantigen (SAg), in particular staphylococcal enterotoxin B1 (SEB-1). Generally a SAg stimulation resulted in a specific activation of destinct vβ T cell subsets. It has been shown, that an activation with SEB-1 resulted in a specific upregulation of LAP expression in TCRs which were directly affected. It is demonstrated herein that LAP was expressed up to 83% upon SAg activation in vβ17 motif. Non-affected TCRs, such as the vβ16 motif, were not been acivated, resulting in a leaked LAP expression in FOXP3+ cells. This demonstrates for the first time that T_{regs} specifically activated by their TCR can be dinstiguished from non-antigen-specific T_{regs} in a complex mixture of cells.

### LAP identifies allogeneic activated Treg subsets and can be used to substantially enrich antigen-specific cells

Based on these findings the identification of allogeneic specific regulatory T cells according to their LAP expression was investigated. Freshly isolated PBMCs were allogeneic activated with unmatched and unrelated PBMCs. With this methodology it has been identified for the first time alloantigen specific peripheral T_{regs} based on their surface expression of LAP. With a ratio of 10-12% those allospecific T_{regs} were capable for their separation.

After separation, allospecific CD4+LAP+ T_{regs} those cells were expanded for two rounds with α-CD3/α-CD28 Dynal beads and IL-2, rested for 48 hours and subsequently presented to a recall antigen. Prior to the re-stimulation the downregulation of LAP in those rested LAP+ T_{regs} was FACS confirmed. According to the LAP expression post-activation with a ratio of 68% upon specific activation by the recall Ag we could confirm the specificity of isolated and expanded LAP+ T_{regs}. A third party reaction of these allospecific LAP+ T_{regs} resulted in a diminished expression of LAP with up to 17%; while the polyspecific activation of these allospecific CD4+LAP+ cells resulted in a LAP expression of up to 89%.

### The marker combination CD4+CD154-LAP+ allows a direct access to Ag specific T_{regs} from complex mixture of cells

Although LAP is a good marker for the identification of antigen-specific T_{regs} CD4+LAP+ population still contained up to 17% of LAP+ cells which could not be assigned to the FOXP3+ population (Figure 1a). The outgrowth of these non-regulators accompanied by the reduced regulative capacity of isolated cells constitutes one major limitation of ex vivo expanded T_{regs} cells. For the use in clinical trials one is aiming for a higher purity of antigen-specific cells. Frentsch et al., reported that CD154 constituted as a good activation marker of recently activated effector T cells (Teffs). The use of the CD 154 signal was hindered by the fact of a rapid internalization after contact to its ligand CD40 on antigen-presenting cells (APCs). However, it was shown that the exertion of α-CD40 prevented the CD40/CD40L interaction and stabilized therefore the CD154 expression (Frentsch, M. et al., Nat Med 11, 1118-1124 (2005).

It was analysed whether the negative selection of CD 154 cells could increase the purity of Ag specific T_{regs}. In this regard it is demonstrated herein that both rested and activated regulatory T cells leaked this marker on their surface (Figure 3a,c), while effector T cells (Teffs) were upregulating CD154 expression rapidly upon stimulation (Figure 3b,d). The FACS data indicated that with the implementation of CD154 a significant increase in the purity of isolated activated regulatory T cells can be achieved. Combining depletion of CD154+ cells with positive selection of LAP expression resulted in a significant higher purity of isolated activated T_{regs} of more than 90%, whereas the selection based on CD4 and LAP resulted in 74% of activated T_{regs}. Similarly, it has been validated that the combination of CD4 and GARP with CD154 caused an increase of GARP+ T_{regs} up to 80% whereas without CD154 negative selection only 71% of activated T_{regs} can be determined. TSDR methylation analysis sustained the flow cytometric data demonstrating the involvement of CD154 marker increased the purity of activated LAP+ T_{regs} (84±1) not only in relation to CD4+LAP+ T_{regs} (72±1), but also compared to the purity of today's Treg gold standard CD4+CD25+CD127low (74±1). Furthermore, it has been confirmed herein, that CD154-, LAP+ T_{regs} represented the best marker combination for the pure isolation of activated T_{regs}. The analysis of CD154-, GARP+ T_{regs} revealed also an access to activated T_{regs.} (Figure 4).

### LAP+ T_{regs} are expandable with a stable FOXP3 expression and with a stable suppressive capacity

Isolated CD4+CD154-LAP+ were cultured and expanded for 21 days. The Treg purity was assessed by the FACS analysis of FOXP3 expression and compared to isolated and expanded CD4+CD25high as well as CD4+CD25+CD127low cells. The data indicated that an outgrowth of FOXP3- cells did not occur in CD4+CD154-LAP+ population. According to the analysis on post expansion 82% of cultured cells kept the FOXP3 expression, while only 18% belonged to the non-regulatory FOXP3- compartment (Figure 5a). Those numbers were comparable with the analysis of CD4+CD25+CD127low cells, where 84% were positive for FOXP3 and 16% constituted the FOXP3- cell fraction (Figure 5b). Importantly, the isolation of CD4+CD25high cells had shown the lowest purity in respect to FOXP3+ T_{regs}. Only 60% provided the Treg fraction, while with a frequency of 40% CD4+CD25high cells showed the largest outgrowth of FOXP3- cells (Figure 5c) although fold expansion of cultured CD4+CD154-LAP+ cells were up to 3 times higher (Figure 5d). Further, it has been demonstrated that expanded CD4+CD154-LAP+ cells possessed the highest suppressive capacity compared to CD4+CD25high and CD4+CD25+CD127low cells (Figures 6a-c).

### CD4+CD154-LAP+ cells prevent a DTH-like swelling response in the trans vivo DTH

The potency of CD4+CD154-LAP+ polyspecific and Ag specific T_{regs} in terms of prevention a graft rejection have been examined and compared with the current standard CD4+CD25high T_{regs} in various models. In the trans vivo Delayed Type Hypersensitivity (DTH) response the potency of CD4+CD25high and of CD154-, LAP+ poly- or Ag specific T_{regs} to attenuate a DTH-like swelling response has been studied. To assess the regulatory capacity of activated T_{regs}, polyspecific stimulated CD4+CD154-LAP+ cells as well as antigen specific LAP+ T_{regs} have been isolated and expanded. Control animals which received mismatched PBMCs from two different donors in equal parts developed a strong DTH reaction. On the basis of this strong reaction the potency of T_{regs} concerning to its inhibition has been assessed. The addition of CD4+CD154-LAP+ T_{regs}, whether poly- or Ag specific, could mitigate this strong DTH reaction while CD4+CD25high T_{regs} showed a mild influence on the swelling response. But it could be shown convincingly that the addition of Ag specific LAP+ T_{regs} could reduce the DTH-like swelling response by up to nine fold, whereas polyspecific LAP+ T_{regs} resulted in a two-fold diminished swelling response (Figure 6).

### CD4+CD154-LAP+ cells prevent a graft rejection in a humanized in vivo model

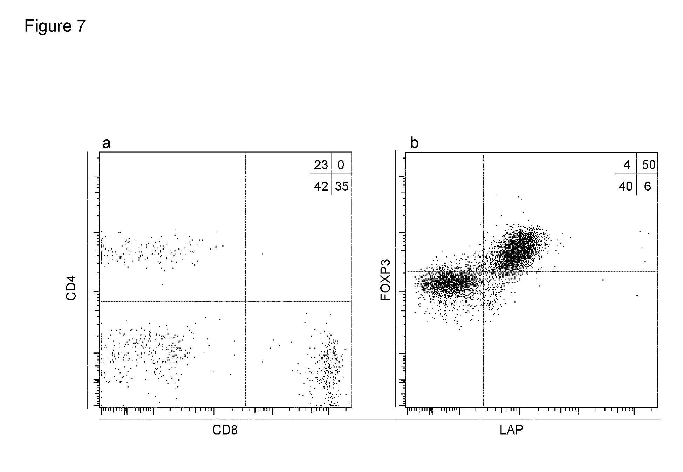

Having shown that allospecific LAP+ T_{regs} were capable to reduce significantly DTH-like swelling response, it is assessed that these cells could play a major role in the regulation of an alloimmune response. The experiments clearly indicated that the humanized mouse model enabled the in vivo monitoring of role and function of allogen specific human regulatory T cells and their impact to induce tolerance against antigenmismatched PBMCs. Therefore we reconstituted *NOD-RAG1^{null}IL2rγ^{null}* mice with human PBMCs and it has been shown that those animals developed a pool of functional human T cells. Our FACS data revealed that the reconstituted human T cells in mice showed a similar phenotype in respect to the expression of FOXP3 and LAP. The polyclonal activation of spleen derived human CD4+ cells resulted in an upregulation of LAP in the FOXP3 + T cell subset (Figure 7).

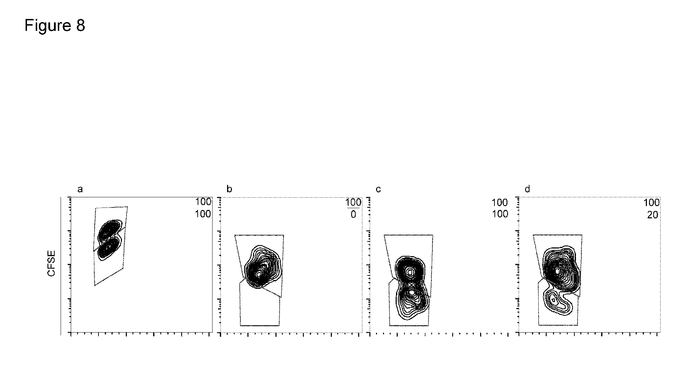

Therefore, the regulation of donor PBMCs in recipient reconstituted NOD-RAG1^{null}IL2rγ^{null} animals have been compared. For the adjacent identification of donor and recipient PBMCs both populations were labelled prior to injection with CFSE in different intensities. According to the trans vivo assay experiments the labelled PBMCs were injected in equal parts into recipient reconstituted mice. The read-out parameter of donor-graft tolerance is the inhibition of donor PBMCs rejection in recipient reconstituted mice. The analysis of murine spleen revealed that three hours post i.p. injection donor PBMCs were fully rejected. To assess the capability for tolerance induction of LAP+ T_{regs} prior to injection polyspecific or rather recipient derived donor specific LAP+ T_{regs} were added to the donor/recipient PBMCs mixture in clinical relevant doses in two different settings. We could demonstrate, that the assessment of polyspecific LAP+ T_{regs} resulted in the modest maintenance of donor PBMCs while the majority of donor PBMCs were rejected. By contrast and in line with the results from the trans vivo DTH assay, a small number of recipient derived donor specific LAP+ T_{regs} were capable to induce tolerance and to prohibit the clearance of donor PBMCs. The FACS data revealed, that three hours post injection both recipient and donor PBMCs could be identified and therefore those allogeneic PBMCs were not killed or rejected (Figure 8).

### Conclusion

Recent protocols for the isolation of T_{regs} are based on the isolation of CD4+CD25high cells resulting in a Treg purity of 40-60%. This suboptimal purity is unlikely to be harmful in patients after to hematopoietic stem cell transplantation (HSCT), where polyspecific T_{regs} can be used. However, polyspecific T_{regs} were so far largely ineffetive in the treatment of autoimmunity and alloimmunity. In the latter disease settings adoptive transfer of T_{regs} was just effective if antige-specific T_{regs} were used. However, antigen-specific T_{regs} need a higher purity of T_{regs}, as non T_{regs} could otherwise develop into effector cells which could destroy the tissue to be protected. (Peters, J.H. et al., PLoS ONE 3, e3161 (2008).

Furthermore, in line with the standards of purity, a further critical obstacle to overcome is the necessity of the therapeutical level of potent regulators. Current clinical protocols provide the enrichment of T_{regs} from a prospective transplant recipient, their ex vivo expansion and the subsequently infusion post transplantation (Sagoo, P. et al., Sci Transl Med 3, 83ra42-83ra42 (2011).

The expansion of isolated T regulators in multiple rounds possess the risk of diminishing the regulative capacity. Isolated CD4+CD25+ cells contain a high number of transiently FOXP3+ and CD25high expressing cells, reverting to effector T cells while the expansion procedure and exacerbate disease (Hippen, K.L. et al., Sci Transl Med 3, 83ra41-83ra41 (2011).

This fact reveals the need for a specific marker combination for the isolation of a pure Treg population and its secure maximization to a therapeutical level.

The epigenetic analysis of CD4+LAP+ and CD4+GARP+ isolated cells revealed the superiority of both in terms of Treg identification over today gold standard CD4+CD25high cells. In addition, it is demonstrates herein, that LAP or GARP expression guaranteed the isolation of a specifically TCR-vβ activated subset of antigen-specific T_{regs} either on SAg stimulated cells, and more importantly on alloactivated PBMCs, ruling out the possibility of a bystander TCR activation. LAP thus opened the possibility for the identification of peripheral T_{regs} with a specificity for a destinct antigen. Various experimental rodent models of transplantation and immune monitoring of transplant patients demonstrated that the implementation of polyspecific T_{regs} in the transplant field may result in the delivery of pan-immunsuppressive effects and could be avoided by the specific suppression of donor-alloreactive effector T cells post-transplantation using allospecific T_{regs}. A further advantage of antigen specific regulatory T cells is the decrease of needed therapeutical level to suppress a graft rejection, since their immunomodulatory effect would be concentrated at the site of alloantigen source and immune activation. This feasibility of CD4+LAP+ T_{regs} or CD4+GARP+ T_{regs} corresponded with current requirements for the T_{regs} in clinical transplantation as one of the main approaches. Nonetheless, the moderate increase of non-regulators in CD4+LAP+ cells could also be detrimental in regard to the therapeutical effect. By the depletion of CD154+ cells a significant increase in purity of isolated T_{regs} can be achieved. While in the past CD154 represents a marker for the identification of Ag specific effector T cells, it is demonstrated herein that the expression on rested as well as on activated T_{regs} leaked. This expression profile of CD154 opened up the possibility to eliminate the unwanted cell fractions during the isolation procedures. Indeed, the epigenetic analyses revealed that the isolation of CD154-, LAP+ T_{regs} resulted in an outperforming purity compared to CD4+LAP+ T_{regs} and to today Treg gold standard CD4+CD25+CD127low. More importantly, the demethylated TSDR of CD154-, LAP+ T_{regs} is with respect to the regulatory phenotype a proof of a stable regulatory T cell population since demethylated TSDR is strongly associated with a stable FOXP3 expression (Floess, S. et al., PLoS Biol. 5, e38 (2007) and ensures no conversion of CD4+CD154-LAP+ cells. These findings corresponded strongly with the reported characteristics of LAP and GARP expression. Furthermore LAP/GARP expression was stringently restricted to FOXP3high cells. This continuous expression of high levels of FOXP3 is necessary not only to maintain Treg cell identity but also to repress Th1/2 cell features. The isolation of alloantigen specific LAP+ T_{regs} and their subsequent expansion revealed a stable FOXP3 expression, expansion capabilities and suppressive capacities. This direct access to antigen specific T_{regs} represent a superior step towards the enrichment of clinical relevant regulatory T cell population. With the involvement of the humanized mouse model a deeper insight into the functionality of allospecific CD154-, LAP+ T_{regs} is possible. The outstanding potency of isolated and expanded allospecific CD4+CD154-LAP+ T_{regs} in their ability to a nearly completed regulation of the DTH like swelling response are demonstrated. The in vitro studies showed clearly, that the separation of CD4+CD154-LAP+ T_{regs} result in the isolation of highly pure activated T_{regs}, with even a higher suppressive capacity compared to CD4+CD25high and CD4+CD25+CD127low T_{regs} post-expansion. These circumstances of the superiority of CD154-, LAP+ T_{regs} had been confirmed in the adequate animal model. The regulation of the DTH like swelling caused by the implementation of allospecific CD154-, LAP+ T_{regs} compared to the moderate regulation caused by CD4+CD25high cells reflects the potency of these regulators in regard to induce tolerance vs. allogeneic cells. Furthermore, allogeneic specific T_{regs} possess a higher capability for the regulation of allogeneic reactions than polyspecific T_{regs}. This tremendous ability to inhibit graft rejections by the addition of CD154-, LAP+ T_{regs} has been shown also in the inhibition of killing of allogeneic target cells in vivo. While those allogeneic targets were efficiently eliminated in immune reconstituted mice, their syngeneic counterparts were not affected. With the addition of CD154-LAP+ T_{regs} it is possible to inhibit the elimination of those allogeneic cells in reconstituted mice. According to the trans vivo DTH assays, the in vivo killing manifest the observation that the addition of T_{regs} with a given antigen specifity are more capabale in the prevention of graft rejection compared to the polyclonal LAP+ T_{regs}. Noteworthy tolerance is achieved in a very immunogenic model of transplantation with allogeneic cells or tissues. As CD4+CD154-LAP+ and CD4+CD154-GARP+ can control such potent immune responses, it is assumed that they are able to control weaker immune responses as seen in autoimmunity and allergy. As there are no humanized models of human autoimmune diseases the latter aspect cannot be experimentally proven in the human system. However, it could be demonstrated in autoimmune mouse models that antigen-specific Tregs can control autoimmunity while polyspecific CD4+CD25+ Tregs had no effect.

## Claims

1. A method for the purification of regulatory T-cells (T_{regs}) comprising
- providing a sample containing T-cells from a mammal; and
- separating T_{regs} from other types of T-cells based on the expression of the marker molecule CD154.

2. The method according to claim 1 further comprising the step of enrichment of T-cells based on the expression of the marker molecule CD4.

3. The method according to any one of claims 1 or 2 further comprising the step of enrichment of T-cells based on the expression of the marker molecule latency-associated peptide (LAP) or glycoprotein-A repetitions predominant (GARP), in particular of LAP.

4. The method according to any one of the preceding claims for purification of antigen-specific T_{regs}.

5. The method according to any one of the preceding claims wherein the T-cells present in the sample have been activated in advance, preferably wherein the T-cells are activated poly-specifically or antigen specific, in particular, wherein the activation is an antigen-specific activation.

6. The method according to any one of the preceding claims wherein the separation comprises contacting the sample with a labelled antibody or antibody fragment that specifically recognizes the marker molecules, preferably wherein the antibody is a monoclonal antibody or a molecule containing a fragment of the monoclonal antibody.

7. The method according to any one of the preceding claims, wherein separation or enrichment is effected by immunological selection techniques, in particular, wherein the immunological selection techniques is at least one of flow cytometry, fluorescence-activated cell sorting (FACS), and magnetic bead based isolation techniques.

8. The method according to any one of the preceding claims wherein the purification of T_{regs} is greater than 80 %, preferably greater than 90 % determined by the TDSR methodology.

9. A method for analyzing a sample or identifying in a sample from a mammal for the presence of T_{regs}, in particular, antigen-specific T_{regs} comprising the step of providing a sample containing T-cells from a mammal and determining T_{regs} based on the expression of the marker molecule CD154 in combination with the marker molecule CD4, particularly preferred, the presence of T_{regs} is analyzed based on expression of CD4, CD154 and LAP and/or GARP.

10. A method for analyzing a sample or identifying in a sample from a mam-malfor the presence of T_{regs}, in particular, antigen-specific T_{regs} comprising the step of providing a sample containing T-cells from a mammal and determining T_{regs} based on the expression of the marker molecule LAP and/or GARP in combination with the marker molecule CD4.

11. An isolated population of cells containing at least 80 % T_{regs}, in particular 80 % of antigen specific T_{regs} when activated with an antigen, obtainable by a method according to any one of claims 1 to 9, preferably, wherein antigen specific T_{regs} are present in an amount of at least 90 % whereby the Tr_{egs} are determined according to TDSR.

12. A kit for isolating T_{regs}, in particular, activated T_{regs}, comprising means for labelling and separating CD154 positive cells; means for labelling and isolating CD4 positive T-cells; means for labelling and isolating T-cells expressing LAP and/or GARP; and, optionally, means for isolating labelled cells.

13. The kit according to claim 12, wherein said means for labelling and isolation are antibodies or compounds consisting of or comprising antibody fragments specifically recognizing the marker molecules.

14. The kit according to claim 12 or 13, wherein the label is at least one of a fluorescence, a magnetic or an isotope label.

15. The kit according to any one of claims 12 to 14, wherein the kit comprises instructions for isolation of T_{regs}, preferably, activated T_{regs}.

16. T_{regs} isolated according to a method of any one of claims 1 to 8 or as claimed in claim 11 for use in preventing or treating an autoimmune disorder in a mammal or for use in preventing or treating graft versus host diseases in a mammal or for preventing or treatment of transplant rejection or graft versus host disease in a mammal or for preventing or treatment of allergic diseases in a mammal.
